Europäisches Patentamt

(19) European Patent Office

Office européen des brevets

(11) Veröffentlichungsnummer: **0 1 1 0 476**
**B1**

(12) **EUROPÄISCHE PATENTSCHRIFT**

(45) Veröffentlichungstag der Patentschrift:
**22.04.87**

(51) Int. Cl.⁴: **A 61 B 6/04,** A 61 B 6/10,
F 16 H 49/00

(21) Anmeldenummer: **83201676.0**

(22) Anmeldetag: **29.11.83**

(54) **Anordnung zur Verschiebung eines Geräteteils an einem Röntgengerät mittels einer endlosen Kette.**

(30) Priorität: **02.12.82 DE 3244578**

(43) Veröffentlichungstag der Anmeldung:
**13.06.84 Patentblatt 84/24**

(45) Bekanntmachung des Hinweises auf die Patenterteilung:
**22.04.87 Patentblatt 87/17**

(84) Benannte Vertragsstaaten:
**BE DE FR GB IT**

(56) Entgegenhaltungen:
**BE - A - 889 159**
**DE - A - 2 205 767**
**DE - C - 352 059**
**US - A - 3 472 085**
**US - A - 3 473 024**
**US - A - 3 588 500**
**US - A - 3 624 398**
**US - A - 4 164 656**

(73) Patentinhaber: **Philips Patentverwaitung GmbH,
Billstrasse 80, D-2000 Hamburg 28 (DE)**
(84) Benannte Vertragsstaaten: **DE**

(73) Patentinhaber: **N.V. Philips' Gioeilampenfabrieken,
Groenewoudseweg 1, NL-5621 BA Eindhoven (NL)**
(84) Benannte Vertragsstaaten: **BE FR GB IT**

(72) Erfinder: **Schmedemann, Walter, Dorfstrasse 80,
D-2000 Tangstedt (DE)**

(74) Vertreter: **Hartmann, Heinrich, Dipl.-Ing. et al, Philips
Patentverwaitung GmbH
Billstrasse 80 Postfach 10 51 49,
D-2000 Hamburg 28 (DE)**

**Beschreibung**

Die Erfindung betrifft eine Anordnung nach dem Oberbegriff des Hauptanspruchs.

Eine derartige Anordnung ist aus der US-PS 3 473 024 bekannt. Dabei ist das Geräteteil eine Tischplatte eines Röntgenuntersuchungsgerätes. Das Kettenrad wird bei Bedarf von einem Motor angetrieben. Ist bei einem derartigen Gerät der Antrieb abgeschaltet, ist das antreibbare Kettenrad blockiert, damit das Geräteteil in der jeweiligen Position bleiben kann.

Bei einer solchen Anordnung wird die Kette durch die auf das Geräteteil einwirkenden Kräfte und zusätzlich noch durch den Motorantrieb belastet. Durch einen Bruch der Kette kann der Patient gefährdet werden. Um die mit einem solchen Bruch verbundenen Gefahren zu vermeiden, existieren gesetzliche Vorschriften (IEC 601-1, 28.3; VDE 0750, Teil 1), die effektiv eine zwölffache statische Sicherheit verlangen, wenn keine zusätzliche Sicherung vorhanden ist, die verhindert, dass bei einem Bruch des Seiles das Geräteteil herabfällt. Das bedeutet also, dass die Kette für das Zölffache der im Normalbetrieb auftretenden statischen Belastung durch das Geräteteil ausgelegt sein muss.

Wenn hingegen eine derartige Fallsicherung vorhanden ist, ist die Situation bei einem Kettenbruch weniger kritisch. In diesem Fall muss die Kette lediglich für die vierfache Höchstlast ausgelegt sein.

Aufgabe der vorliegenden Erfindung ist es, eine Anordnung der eingangs genannten Art so auszubilden, dass bei einem Kettenbruch das Geräteteil sich nur geringfügig bewegen kann. Diese Aufgabe wird erfindungsgemäss durch die im Kennzeichen des Hauptanspruchs angegebenen Massnahmen gelöst.

Die starre Führung umschliesst die Kette allseits so, dass sie sich in seitlicher Richtung, d.h. in Richtung senkrecht zur Tangente an die Kette im betreffenden Punkt nur geringfügig bewegen kann. Wenn die Kette bricht – und dieser Bruch findet in der Regel in dem auf Zug beanspruchten Teil der Kette statt –, übt daher das Geräteteil auf den anderen Teil der Kette einen Druck aus, dem die Kette jedoch kaum seitlich ausweichen kann, weil sie durch die Führung daran gehindert wird. Infolgedessen nimmt die Kette die Druckkraft auf und hält das Geräteteil praktisch in der Stellung fest, in der es sich vor dem Reissen der Kette befand.

An dieser Stelle sei erwähnt, dass aus der BE-A 889 159 bereits eine Einrichtung zur Drehung des Streustrahlenrasters bei einem Röntgenschichtaufnahmegerät bekannt ist, bei dem das Streustrahlenraster in einem kreisförmigen Träger angeordnet ist, der von einer mit ihm fest verbundenen Kette umschlossen wird. Der Träger mit der Kette befindet sich in einem Grundrahmen, in dem sich auch ein in die Kette eingreifendes Zahnrad befindet, das von einem Motor angetrieben wird. Auch hierbei kann die Kette nur geringfügige seitliche Bewegungen ausführen. Dies ist deshalb erforderlich, weil die Kette in Verbindung mit dem Träger wie ein Zahnrad wirken soll. Eine Verschiebung eines Geräteteils in einer Richtung ist mit dieser Konstruktion nicht möglich, und es geht dabei auch nicht um die Sicherheit im Falle eines Kettenbruchs.

Grundsätzlich kann die Führung aus mehreren Teilen bestehen, die das antreibbare Kettenrad – sowie ein weiteres zum Umlenken der Kette erforderliches Kettenrad – und die zwischen den Kettenrädern liegenden Teile der Kette einzeln umschliessen. Eine bevorzugte Weiterbildung der Erfindung sieht jedoch vor, dass die Führung als Hohlprofilschiene mit Ausnehmungen für die Kettenräder und mit Kanälen für die Ketten ausgebildet ist. In weiterer Ausgestaltung dieser Ausführungsform ist bei einem Röntgengerät, bei dem das Geräteteil die Tischplatte ist, vorgesehen, dass die Hohlprofilschiene der Führung der Tischplatte dient. Die Hohlprofilschiene hat hierbei also zwei Funktionen: Sie dient einerseits der Führung der Tischplatte und sichert andererseits zusammen mit der Kette die Tischplatte gegen Herabfallen.

Die Erfindung wird nachstehend anhand der Zeichnung näher erläutert. Es zeigen:

Fig. 1 ein Röntgengerät, bei dem die Erfindung anwendbar ist,

Fig. 2 einen Querschnitt durch eine als Hohlprofilschiene ausgebildete Führung in Richtung senkrecht zur Kette, und

Fig. 3 einen Querschnitt durch diese Führung in Richtung parallel zur Kettenebene.

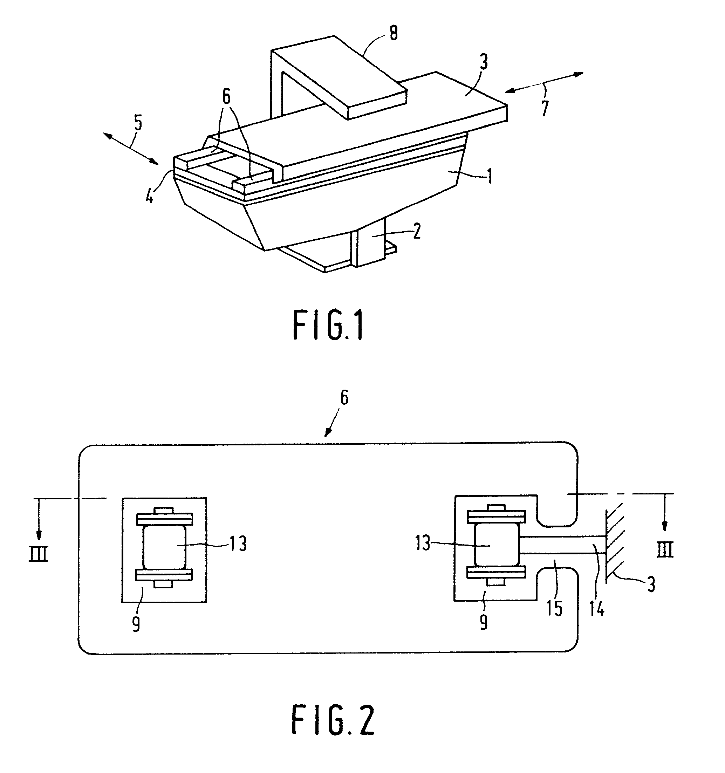

In Fig. 1 ist ein Röntgenuntersuchungsgerät dargestellt, dessen Rahmen 1 um eine horizontale Achse schwenkbar in einer Fusskonstruktion 2 gelagert ist. Der Rahmen trägt einen parallel zur Schwenkachse, d.h. in Richtung des Doppelpfeils 5, verschiebbaren Querwagen 4, der mit zwei parallelen Führungsschienen 6 versehen ist, die senkrecht zur Schwenkachse verlaufen und zur Führung einer Tischplatte 3 in deren Längsrichtung, d.h. in Richtung des Doppelpfeiles 7, dienen. Ausserdem umfasst das Röntgenuntersuchungsgerät noch ein Röntgenzielgerät 8.

Die Verschiebung der Tischplatte 3 erfolgt mit Hilfe einer in Fig. 1 nicht näher dargestellten Kette, auf die über ein Kettenrad ein Motor – beides ebenfalls nicht dargestellt – einwirkt. Wenn der Motor abgeschaltet ist, ist das von ihm angetriebene Kettenrad blockiert – beispielsweise, weil der Motor über ein selbsthemmendes Getriebe mit dem Kettenrad gekoppelt ist oder weil er als Bremsmotor ausgeführt ist –, so dass die Tischplatte ihre erreichte Position beibehält, und zwar auch dann, wenn sie senkrecht steht und durch den Patienten, z.B. über eine Fussbank, belastet wird. Insoweit ist das Röntgenuntersuchungsgerät bekannt.

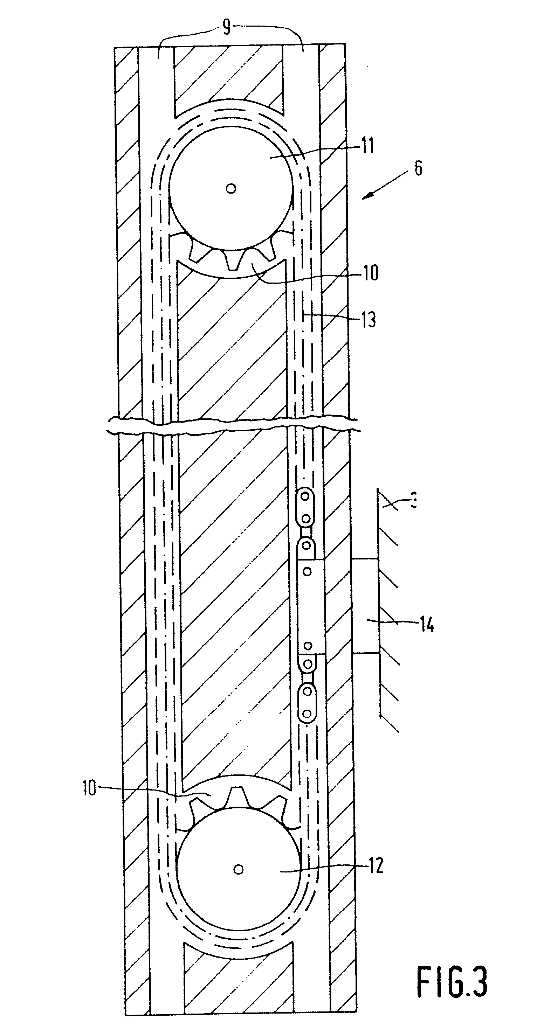

In den Figuren 2 und 3 sind Querschnitte einer Führungsschiene 6 dargestellt, und zwar in Richtung senkrecht zur Tischplatte (Fig. 2) und in einer zur Tischplate parallelen Ebene (Fig. 3). Die Führungsschiene ist als Hohlprofilschiene mit zwei in Schienenlängsrichtung verlaufenden Kanälen 9 mit rechteckigem Querschnitt ausgebildet. In der Nähe der Enden der Führungsschiene sind zylinderförmige Ausnehmungen 10 vorgesehen, in denen je ein Kettenrad 11 bzw. 12 läuft, wobei das Kettenrad 12 mit einem nicht näher dargestellten Antriebsmotor gekoppelt ist. Um die beiden Kettenräder ist eine Rollenkette 13, z.B. nach DIN 8187, geführt, die in den Kanälen 9 verläuft; in der Zeichnung sind nur wenige Glieder dieser Kette angedeutet. Mit dieser Rollenkette ist eine Kopplungseinrichtung 14 verbunden, die aus einem in Längsrichtung der Schiene verlaufenden Schlitz 15 herausragt und mit der einen Seite der Tischplatte 3 verbunden ist. Die Abmessungen des Schlitzes sind kleiner als die Abmessungen eines Kettengliedes, so dass die Kette unter keinen Umständen seitlich aus dem Schlitz heraustreten kann. Wie die Figuren 2 und 3 zeigen, sind die Abmessungen der Ausnehmungen 10 und der Kanäle 9 so auf die Abmessungen der Kettenräder 11 und 12 und der Kette 13 abgestimmt, dass die Kette überall in geringem Abstand (von z.B. 1 mm) an den sie umschliessenden Wänden des Hohlprofils vorbeiläuft.

Wenn das Röntgenuntersuchungsgeät in eine vertikale Stellung geschwenkt ist, so dass die Führungsschiene 6 eine Lage einnimmt, in der sich das antreibbare Kettenrad 12 am unteren Ende der Schiene befindet, wirkt das Gewicht der Tischplatte (und des darauf befindlichen Patienten) über die Kopplungsvorrichtung 14 auf die Kette 13 ein. Der zwischen der Kopplungsvorrichtung 14 und dem der Umlenkung dienenden Kettenrad befindliche Teil sowie der (linke) die beiden Kettenräder direkt miteinander verbindende Teil werden dabei auf Zug beansprucht, während der verbleibende Teil (zwischen der Koppelvorrichtung 14 und dem antreibbaren Kettenrad 12) nur entsprechend der Vorspannung der Kette belastet wird. Wenn nun der auf Zug belastete Teil der Kette bricht, wird dieser Teil der Kette entlastet, während der restliche Teil der Kette auf Druck beansprucht wird. Die Kette kann diesem Druck aufgrund der engen Führung in dem Kanal 9 bzw. in der Ausnehmung 10 seitlich (d.h. auch in Richtung senkrecht zur Zeichenebene der Fig. 3) nicht ausweichen. Da, wie in Verbindung mit Fig. 1 bereits erläutert, das antreibbare Kettenrad 12 entweder blockiert ist bzw. sich nur mit der durch den eingeschalteten Motor bestimmten Winkelgeschwindigkeit drehen kann, wird der zwischen der Kopplungseinrichtung 14 und dem antreibbaren Kettenrad 12 befindliche Teil der Kette auf Druck beansprucht, wobei die Tischplatte 3 nicht nach unten fallen kann, sondern sich nur wenig bzw. entsprechend der Motordrehzahl bewegt.

Die Tischplatte kann somit bei einem Kettenbruch nicht herabfallen. Nach den gesetzlichen Bestimmungen muss die Kette 13 daher nur für das Vierfache der statischen Höchstlast ausgelegt sein; sie kann also verhältnismässig dünn sein.

Die zweite Führungsschiene kann entsprechend spiegelsymmetrisch angeordnet sein, so dass die Schlitze in den beiden Schienen von der jeweils anderen Schiene abgewandt sind. Die Kettenräder müssen dann mit der gleichen Drehzahl, aber mit entgegengesetztem Umlaufsinn angetrieben werden.

Die Kette 13 muss nicht fest mit der Tischplatte 3 verbunden sein; sie kann über eine Zahnstange, die fest mit der Tischplatte verbunden ist und deren Zähne in die Kette eingreifen mit dieser gekoppelt sein.

## Patentansprüche

1. Anordnung zur Verschiebung eines Geräteteils an einem Röntgengerät mittels einer endlosen Kette, die über Kettenräder geführt ist, von denen eines antreibbar ist, dadurch gekennzeichnet, dass die Kette (13) im Innern einer starren in der Verschiebungsrichtung relativ zum Röntgengerät ortsfesten Führung (6) verläuft, die die Kettenräder (11, 12) und die zwischen den Kettenrädern befindlichen Teile der Kette umschliesst, so dass die Kette allseits nur geringfügige seitliche Bewegungen ausführen kann und die einen Schlitz (15) aufweist, durch den hindurch die Kette mit dem Geräteteil (3) gekoppelt ist.

2. Anordnung nach Anspruch 1, dadurch gekennzeichnet, dass die Führung als Hohlprofilschiene (6) mit Ausnehmungen (10) für die Kettenräder (11, 12) und mit Kanälen (9) für die Kette (13) ausgebildet ist.

3. Anordnung nach Anspruch 2, wobei das Geräteteil die Tischplatte eines Röntgengerätes ist, dadurch gekennzeichnet, dass die Hohlprofilschiene (6) der Führung der Tischplatte (3) dient.

## Claims

1. A device for the displacement of a part of an X-ray apparatus by means of an endless chain which is guided by sprockets, one of which is drivable, characterized in that the chain (13) is accomodated inside a rigid guide (6) which is fixed in the displacement direction relative to the X-ray apparatus, said guide enclosing the sprockets (11, 12) and the parts of the chain situated between the sprockets, so that the chain is limited in its freedom of movement in the lateral direction on all sides, and being provided with a slot (15) wherethrough the chain is coupled to the part (3) of the apparatus.

2. A device as claimed in Claim 1, characterized in that the guide is constructed as a rail (6) having a hollow profile with recesses (10) for the sprockets (11, 12) and ducts (9) for the chain (13).

3. A device as claimed in Claim 2 where the part of the apparatus is formed by the table-top of an X-ray apparatus, characterized in that the rail (6) comprising the hollow profile serves to guide the table-top (3).

**Revendications**

1. Dispositif de déplacement d'une partie d'appareil d'un appareil radiologique au moyen d'une chaîne sans fin qui est guidée par l'intermédiaire de pignons dont l'un peut être entraîné, caractérisé en ce que la chaîne (13) se déplace à l'intérieur d'un guide (6) fixé rigidement dans le sens du déplacement par rapport à l'appareil radiologique, qui enveloppe les pignons (11, 12) et les parties de la chaîne se trouvant entre les pignons, de sorte que de tous côtés la chaîne ne peut effectuer que de faibles déplacements latéraux et qui présente une fente (15) à travers laquelle la chaîne est couplée à la partie d'appareil (3).

2. Dispositif suivant la revendication 1, caractérisé en ce que le guide a la forme d'un rail profilé creux présentant des évidements (10) pour les pignons (11, 12) et des canaux (9) pour la chaîne (13).

3. Dispositif suivant la revendication 2, dans lequel la partie d'appareil est la table d'un appareil de radiologie, caractérisé en ce que le rail profilé creux (6) sert à guider la table (3).

FIG.1

FIG.2

0 110 476

FIG.3